# FASCICULE DE BREVET EUROPEEN

(11) **EP 1 699 423 B1**
(45) Date de publication et mention de la délivrance du brevet: **07.11.2007**
(21) Numéro de dépôt: 04805628.7
(22) Date de dépôt: 03.12.2004
(51) Int. Cl.: A61K 8/18, A61Q 19/00, A61Q 19/02, A61K 36/00

(54) **UTILISATION D'UN LYOPHILISAT DE CELLULES VEGETALES DEDIFFERENCIEES AFIN DE DEPIGMENTER ET/OU D'ECLAIRCIR LA PEAU**
VERWENDUNG EINES LYOPHILISATS AUS ENTDIFFERENZIERTEN PFLANZENZELLEN FÜR DIE DEPIGMENTIERUNG UND/ODER BLEICHUNG DER HAUT
USE OF A LYOPHILISATE OF DEDIFFERENTIATED PLANT CELLS FOR SKIN DEPIGMENTATION AND/OR LIGHTENING

(30) Priorité: 29.12.2003 FR 0315521
(43) Date de publication de la demande: 13.09.2006
(73) Titulaire: BiotechMarine, 22260 Pontrieux (FR)
(72) Inventeur: MEKIDECHE, Nicole, F-22620 Ploubazlanec (FR)
(74) Mandataire: Lepeudry-Gautherat, Thérèse
(86) Numéro de dépôt international: PCT/FR2004/003109
(87) Numéro de publication internationale: WO 2005/072697

(56) Documents cités:
- EP-A- 1 064 932
- WO-A-01/82887
- WO-A-03/077881
- FR-A- 2 657 011
- FR-A- 2 846 242

## Description

Utilisation dans une composition cosmétique ou pharmaceutique d'au moins un lyophilisat de cellules végétales dédifférenciées, lesquelles sont des cellules de plantes halophiles, afin de dépigmenter et/ou éclaircir, de protéger et de régénérer l'épiderme.

L'invention concerne l'utilisation d'au moins un lyophilisat de cellules végétales dédifférenciées, lesquelles sont des cellules de plantes halophiles, dans une composition cosmétique ou pharmaceutique à des fins de dépigmentation et/ou éclaircissement de l'épiderme accompagné d'un effet protecteur et régénérateur. L'invention a, de plus, pour objet une composition cosmétique ou pharmaceutique à usage topique comprenant au moins un tel lyophilisat.

La peau est une enveloppe protectrice qui représente l'interface entre le milieu extérieur et les autres organes internes. Elle n'est toutefois pas hermétique et, en tant qu'organe d'absorption, elle permet aux substances dissoutes de pénétrer par l'intermédiaire des pores et des follicules pileux.

La peau protège contre le froid, la chaleur, le rayonnement ; contre la pression, le frottement ; contre les lésions chimiques ; contre la pénétration des micro-organismes ; de la perte d'eau et de chaleur.

La protection naturelle de la peau contre le rayonnement ultraviolet passe par un pigment brun, la mélanine. La mélanine est présente dans des cellules des couches basales de l'épiderme : les mélanocytes. Elle est synthétisée à partir d'un acide aminé, la tyrosine, modifié par une enzyme, la tyrosinase. La synthèse de la mélanine est induite par les rayons UV. La coloration foncée de la peau est une première protection naturelle contre le soleil, qui est bien insuffisante pour les peaux les plus claires. Le bronzage est la conséquence, sous l'action des UV, de l'augmentation de l'activité mélanocytaire de synthèse de la mélanine et du stockage de la mélanine dans les kératinocytes.

La quantité de mélanine et le nombre de mélanocytes sont des facteurs programmés génétiquement qui donnent sa couleur à la peau.

Une production excessive et localisée de mélanine provoque l'apparition de taches : taches de rousseur, masque de grossesse, lentigos solaires ou séniles, etc... En effet, selon les dernières nomenclatures, les taches de vieillesse sont les lentigos séniles et les lentigos solaires sont les autres taches.

Les "taches de vieillesse" sont de petites taches brun pâle, planes et généralement rondes, le plus souvent sur le visage, le dos des mains, le décolleté, les avant-bras. Elles sont provoquées par la combinaison du soleil et du vieillissement. Leur nombre augmente avec l'âge. Sous l'action répétée des rayons UV, les mélanocytes finissent par produire trop de mélanine.

Les lentigines sont des "taches de rousseur" qui ne disparaissent pas en hiver, brunes, minuscules et nombreuses, très proches les unes des autres. On les trouve le plus souvent sur le visage, les épaules et le décolleté. Elles sont provoquées par une overdose d'ultraviolets et peuvent se former dès le plus jeune âge.

Le "masque de grossesse" se manifeste par de grandes taches brunes au contour irrégulier, le plus souvent sur le visage, qui prennent parfois l'aspect d'un masque. Quand ces taches apparaissent au cours de la grossesse, on parle de chloasma, sinon on parle de mélasma. Elles sont provoquées par une stimulation hormonale (grossesse, hormonothérapie) combinée à l'exposition aux rayons UV.

Tous ces types de taches peuvent également être liés au patrimoine génétique et donc à l'hérédité.

Devant l'aspect disgracieux de ces surconcentrations de mélanine, il est d'un intérêt particulièrement important d'avoir accès à des préparations à usage topique qui permettraient de prévenir leur apparition et/ou de les atténuer.

Les fabricants de préparations cosmétiques et pharmaceutiques sont constamment à la recherche de principes actifs non agressifs permettant d'inhiber ou de bloquer la synthèse de la mélanine, directement ou indirectement, ou d'inhiber ou de bloquer le transfert des mélanosomes aux kératinocytes, et donc d'éclaircir ces taches tout en protégeant ces zones d'une pigmentation solaire. De même, les fabricants de préparations cosmétiques cherchent des principes actifs dépigmentants non agressifs devant le désir croissant des populations noires et asiatiques d'éclaircir leur peau, tout en leur proposant un produit protecteur de la peau.

Pour pallier aux désagréments des autres traitements disponibles fastidieux et/ou agressifs (laser, cryothérapie) de dépigmentation, ces principes actifs doivent en même temps protéger la peau et/ou stimuler la régénération des cellules la constituant.

En effet, la protection de la peau et/ou la stimulation de la régénération des cellules de la peau permettent de lutter en même temps contre un facteur aggravant des taches : le vieillissement cutané aux multiples causes.

La première cause du vieillissement cutané est le vieillissement « programmé » qui peut être accéléré par le stress, le tabagisme et certaines maladies. Avec les années, la peau perd de son élasticité car le derme produit toujours moins de fibres de collagène et d'élastine. D'où l'affaiblissement progressif du tissu conjonctif et le relâchement de la peau. La capacité de renouvellement de l'épiderme tend également à diminuer, celui-ci devient plus sec et plus mince car son métabolisme est altéré. La peau prend également avec le temps un aspect grisonnant qui donne un teint terne contre lequel un soin éclaircissant peut aussi lutter.

La deuxième cause du vieillissement est la diminution de la production hormonale qui entraîne la diminution progressive des fonctions tissulaires, cellulaires et organiques. Les hormones telles que l'hormone de croissance (HGH), la testostérone, la DHEA et la mélatonine sont produites en grandes quantités jusqu'à l'âge de 20 ans et elles favorisent le renouvellement cellulaire.

La conséquence de ces différentes causes du vieillissement conjuguées aux effets de l'environnement (pollutions diverses : gaz d'échappement, fumées de cigarettes, fumées d'usines, produits chimiques...) amènent à la surproduction de radicaux libres qui ont pour cibles les différents composants de la cellule : protéines, lipides, sucres et l'ADN et qui constituent ainsi une autre cause du vieillissement cutané. Certaines influences extérieures les poussent à entrer en réaction car ils recherchent constamment d'autres molécules avec lesquelles ils peuvent se lier. Ils attaquent alors les fibres de collagène, les membranes cellulaires et la couche graisseuse de la peau. Ils altèrent le patrimoine génétique des cellules, de sorte que la qualité des nouvelles cellules de la peau diminue.

Le corps se protège contre ces agresseurs par des systèmes enzymatiques s'opposant à ces réactions d'oxydation (antioxydants). Mais dès l'âge de vingt ans, les mécanismes de défense naturels s'affaiblissent progressivement, de sorte que la peau ne peut plus se défendre toute seule.

La Demanderesse a découvert de manière surprenante et inattendue qu'un lyophilisat de cellules végétales dédifférenciée, lesquelles sont des cellules de plantes halophiles, permet d'atteindre cette combinaison d'effets recherchée : il dépigmente et/ou éclaircit l*'*épiderme avec une parfaite innocuité tout en le protégeant et le régénérant.

Les cellules dédifférenciées conservent toutes les potentialités cellulaires comme les cellules souches. Elles expriment tous les gènes de leur génome donc toutes les protéines qui permettent à chaque type de cellule spécialisée de se protéger du milieu extérieur.

L'invention a donc pour objet premier l'utilisation dans ou pour la fabrication d'une composition cosmétique ou pharmaceutique d'au moins un lyophilisat de cellules végétales dédifférenciées, lesquelles sont des cellules de plantes halophiles, ledit lyophilisat permettant de dépigmenter et/ou éclaircir, de protéger et de régénérer l'épiderme.

On entend par "cellule végétale dédifférenciée" toute cellule végétale ne présentant aucun caractère de spécialisation et pouvant régénérer à elle seule un plant entier de végétal dont elle provient. Elle peut être isolée à partir de tout échantillon de plante entière ou d'organe comme les feuilles, les tiges, les racines, les graines, les fleurs, les pétales, les anthères, les fruits, etc... appelé explant.

De manière préférée, on utilise un morceau de feuille ou une graine en tant qu'explant.

On préfère tout particulièrement cultiver l'explant *in vitro*. On entend par "culture *in vitro"* l'ensemble des techniques de l'art antérieur connues de l'homme du métier qui permettent dans des conditions parfaitement contrôlées de régénérer un organe ou une plante entière à partir d'un explant cultivé dans ou sur un milieu nutritif défini. Ces conditions parfaitement maîtrisées permettent une parfaite reproductibilité et homogénéité des plants. En particulier, cette méthode de culture fournit des clones identiques à l'infini. Parmi les méthodes et milieux de culture in vitro décrits dans l'art antérieur, on peut citer à titre d'exemples les milieux de Gamborg (1968), de Murashige et Skoog (1962), de Morel (1970), etc..dont la formulation est décrite dans "Plant Culture Media : formulations and uses" de E.F. George, DJM Puttock et H.J. George (Exegetics Ltd 1987).

En particulier, on préfère utiliser des cellules végétales dédifférenciées de végétal halophile comme les espèces Salicornia ramossisima (Salicorne), Sueda vera, Beta maritima, Obione portulacoides, Armeria maritima, Crithmum maritimum (Criste Marine), Ophrys sphegodes, Artemia vulgaris, Muscaris comosum, Eryngium maritimum, Sanguisorba minor, Cochlearia officinalis, Fumaria officinalis, Vincetoxicum fullonum, Dipsacus fullonum, Heracleum spondylium, Inula crithmoides, Inula brittanica, Inula viscosa, tout particulièrement des cellules végétales dédifférenciées de Criste Marine (Crithmum maritimum). Les végétaux halophiles, également nommés halophytes, sont des végétaux qui tolèrent des sols riches en sel. Ils ont développé des systèmes de défense contre le milieu extérieur agressif qu'ils colonisent. En particulier, les halophytes sont des végétaux du bord de mer capables de supporter un sol riche en sel, l'humidité et le vent. Ils luttent en permanence pour maintenir la pression osmotique dans leurs cellules, l'eau ayant tendance à traverser la membrane plasmique vers le compartiment extracellulaire le plus sodé.

Dans le cas de l'utilisation de végétal halophile, il est particulièrement important de développer une culture in vitro de cellules qui fournit une biomasse infinie et reproductible pour protéger ces espèces, les halophytes étant en danger face à la pollution des mers.

Dans une réalisation particulière de l'invention, il est également possible de modifier certaines conditions de culture (pH, température, composition gazeuse ambiante, composition du milieu de culture, luminosité). Les cellules dédifférenciées tendront alors à produire plus ou moins de certaines substances intracellulaires.

Le second objet de l'invention est une composition cosmétique ou pharmaceutique à usage topique caractérisée en ce qu'elle comprend, dans une base physiologiquement acceptable, 0,05 à 2%, de préférence 0,1 à 1%, de manière particulièrement préférée 0,5% d'au moins un lyophilisat tel que décrit précédemment.

En effet, un tel lyophilisat peut être utilisé comme seul principe actif de la composition selon l'invention. Cependant, plusieurs lyophilisats peuvent être ajoutés à la base de la composition selon l'invention.

Dans un premier mode de réalisation particulier de l'invention, on utilise au moins un lyophilisat de cellules végétales dédifférenciées, lesquelles sont des cellules de plantes halophiles, pour la fabrication d'une composition cosmétique ou pharmaceutique, destinée à rajeunir l'aspect de la peau.

Dans un deuxième mode de réalisation particulier de l'invention, on utilise au moins un lyophilisat de cellules végétales dédifférenciées, lesquelles sont des cellules de plantes halophiles, pour la fabrication d'une composition cosmétique ou pharmaceutique destinée au traitement des taches appelées lentigos.

Dans un troisième mode de réalisation particulier de l'invention, on utilise au moins un lyophilisat de cellules végétales dédifférenciées, lesquelles sont des cellules de plantes halophiles, pour la fabrication d'une composition cosmétique ou pharmaceutique, destinée à éclaircir les peaux noires ou asiatiques.

Les exemples suivants illustrent l'invention sans toutefois en limiter la portée.

### Légende des Figures

- Figure 1 :: morphologie générale, coloration à l'hématoxyline/éosine (HES) sur épiderme reconstitué SKINETHIC^{®} simple (kératinocytes)
- Figure 2 :: morphologie générale, coloration à l'hématoxyline/éosine (HES) sur épiderme reconstitué SKINETHIC^{®} intégrant des mélanocytes
- Figure 3 :: marquage de la filaggrine
- Figure 4 :: marquage de KI-67 (évaluation de l'index mitotique)

### Exemple 1: Culture in vitro de cellules dédifférenciées de Criste Marine à partir de tissu végétal:

### 1- Obtention de cals primaires

On prélève à l'aide de ciseaux des morceaux de tissus dans la zone choisie (3 cm minimums) tige, feuilles... A partir de ce stade de la manipulation, tout doit se dérouler en atmosphère stérile sous une hotte à flux laminaire.

Pour stériliser le matériel végétal, on immerge les tissus 30 s dans l'éthanol puis on élimine le solvant, on les rince par 3 x 100 ml d'H₂O stérile, on les immerge 15 min. dans l'hypochlorite de sodium additionné de quelques gouttes de Tween 20 et on les rince par 3 x 100 ml d'H₂O stérile.
Pour mettre les tissus en culture, on dépose les fragments de tissus dans une boîte de Pétri stérile (125 mm), on découpe des fragments de tissus (2 à 3 mm) en prenant soin d'éliminer les parties blanchies par l'eau de javel. Les explants ainsi obtenus sont légèrement incisés et déposés à demi-enfoncés dans le milieu nutritif gélosé (Tableau 1).

### 2- Repiquage des cals

A ce stade de la manipulation, tout doit se dérouler en atmosphère stérile sous une hotte à flux laminaire.
On prélève au niveau du cal 2 à 3 amas cellulaires (1 à 2 cm) à l'aide d'une spatule,

On dépose et répartit ces amas sur le milieu neuf.

**Composition du milieu de culture solide : Tableau 1**

| Macroéléments | mg/l |
|---|---|
| KNO₃ | 2500 |
| (NH₄)₂SO₄ | 134 |
| CaCl₂, 2H₂O | 150 |
| NaH₂PO₄, 2H₂O | 300 |
| MgSO₄, 7H₂O | 250 |

| Microéléments | mg/l |
|---|---|
| MnSO₄, H₂O | 16,9 |
| ZnSO₄, 7H₂O | 8,6 |
| H₃BO₃ | 6,2 |
| Kl | 0,83 |
| Na₂MOO₄, 2H₂O | 0,25 |
| CuSO₄, 5H₂O | 0,025 |
| FeSO₄, 7H₂O | 27,8 |

| Vitamines | mg/l |
|---|---|
| myo-inositol | 100 |
| acide nicotinique | 1 |
| D(+)-panthoténate de calcium | 1 |
| (+)-biotine | 0,01 |
| chlorhydrate de pyridoxal | 1 |
| dichlorure de thiamine | 1 |

| Composés organiques | g/l |
|---|---|
| saccharose | 30 |

| Phytohormones | mg/l |
|---|---|
| acide naphtalène acétique | 1,5 |
| acide 2,4 dichloro-phénoxyacétique | 0,5 |
| kinétine | 0,5 |

| Agent gélifiant | g/l |
|---|---|
| agar | 9 |

### 3- Expansion des cellules du cal en milieu liquide

### • Inoculum d'entretien:

Les cellules du cal sont transférées dans un milieu liquide identique au milieu solide sans agar (Tableau 1). Elles sont cultivées sous agitation (110 tours/min), à 25°C en lumière blanche continue (3500 lux, tubes fluorescents "lumière du jour") dans des erlenmeyers de 250 mL, à raison de 50 mL par erlenmeyer.
Elles sont diluées tous les 10 à 11 jours au 1/4 c'est-à-dire 100 mL dans 400 mL.

### • Production de matière sèche :

Les cellules sont cultivées à partir d'une dilution au 1/4 de l'inoculum d'entretien dans des erlenmeyers de 5L, à raison de 2L de culture par erlenmeyer, sous agitation (110 tours/min), à 25°C en lumière blanche continue (3500 lux, tubes fluorescents "lumière du jour") et ce pendant 12 à 13 jours.

Il faut noter que l'acide 2,4 dichloro-phénoxyacétique est entièrement métabolisé et qu'il ne sera pas retrouvé dans le produit final.

### Exemple 2 Préparation d'un lyophilisat de cellules dédifférenciées de Criste Marine :

La culture cellulaire liquide en suspension est centrifugée pour culoter les cellules.

Les cellules sont passées sur un tamis de 150 à 200 µm, congelées puis lyophilisées dans un lyophilisateur à plaques.

### Exemple 3 : Mise en évidence sur épiderme reconstitué SKINETHIC^{®} de l'absence de toxicité d'une réparation cosmétique contenant un lyophilisat de cellules dédifférenciées de Criste Marine

L'épiderme reconstitué SKINETHIC^{®} est un modèle d'épiderme humain développé et commercialisé par la Société SkinEthic Laboratories (Nice, France).

### 1- Sur épiderme reconstitué SKINETHIC^{®} simple (constitué uniquement de kératinocytes):

Des kératinocytes d'origine humaine sont ensemencés sur des filtres en polycarbonate de 0,63 cm² dans un milieu défini (MCDB 153 modifié) et supplémenté. Les cellules sont cultivées pendant 14 jours à l'interface air/liquide, le milieu de culture étant changé tous les deux jours.

Les épidermes ainsi formés ont été utilisés pour la réalisation de l'étude à partir du 17ème jour de culture.

Un essai préliminaire a été effectué afin de déterminer le temps de contact et la quantité de produit appliqué sur l'épiderme reconstitué n'entraînant pas de cytotoxicité.
Tous les tests ont été conduits en duplicate avec :
Lot 1: épidermes témoins ne recevant pas de produit
Lot 2 : épidermes traités recevant crème PXTS + 0,1% AK205
Lot 3 : épidermes traités recevant crème PXTS + 0,5% AK205
Lot 4 : épidermes traités recevant le produit lyophilisat de cellules (AK 205)
AK205 = lyophilisat de cellules dédifférenciées de Criste Marine obtenu selon les Exemples 1 et 2.
PXTS = pour peaux très sèches

Les épidermes fixés dans une solution formaldéhyde 10% ont été inclus dans des blocs en paraffine. Les coupes verticales de 4 microns ont été colorées à l'hématoxyline/éosine et photographiées sous un microscope optique.

Les cultures doivent présenter des couches cellulaires basales, spineuses, granuleuses et cornées intactes, orthokératosiques, et la stratification épidermique doit être régulière et normale. Les cellules de la couche basale doivent être polarisées verticalement. De nombreux grains de kératohyaline doivent être visibles (en violet) dans la couche granuleuse juste sous la couche cornée.

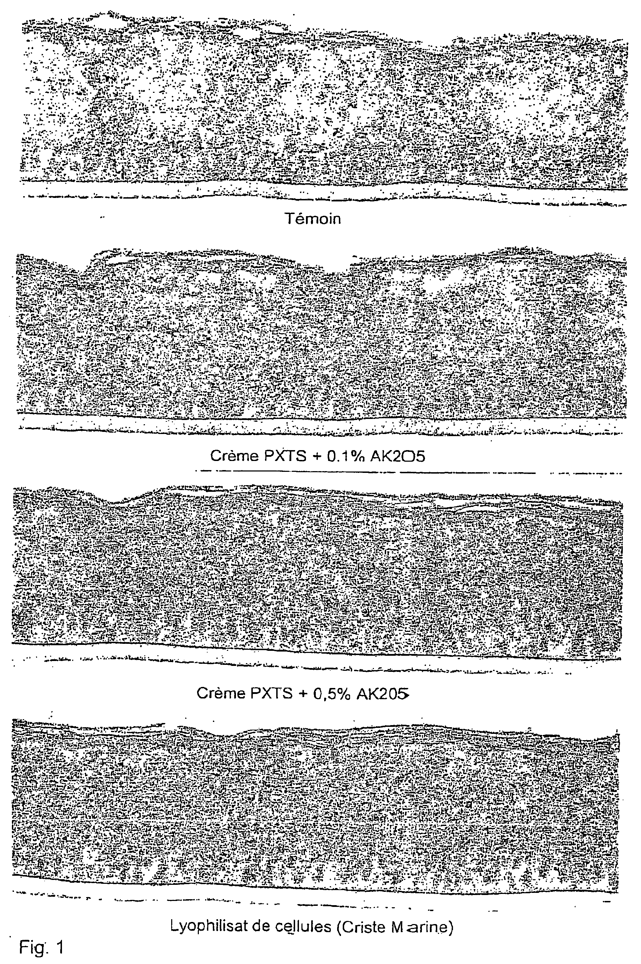

Les produits, crème PXTS+0,1% AK205, crème PXTS+0,5% AK205 et le produit lyophilisat de cellules de Criste Marine (AK 205) déposés à raison de 2 µL par cm², sur des épidermes reconstitués traités pendant 24 heures, comparativement à des épidermes témoins, n'ont induit aucune toxicité. Les images histologiques, après coloration hématoxyline/éosine, d'épidermes traités sont comparables à celles des épidermes témoins (cf. Figure 1).

### 2- Sur épiderme reconstitué SKINZTHIC^{®} intégrant des mélanocytes :

Des kératinocytes d'origine humaine et des mélanocytes sont ensemencés sur des filtres en polycarbonate de 0,63 cm² dans un milieu défini (MCDB 153 modifié) et supplémenté. Les cellules sont cultivées pendant 10 jours à l'interface air/liquide, le milieu de culture étant changé tous les jours.

Les épidermes de type VI (= négroïde) ainsi formés ont été utilisés à partir du 10ème jour de culture.

Un essai préliminaire a été effectué afin de déterminer le temps de contact et la quantité de produit appliqué sur l'épiderme reconstitué n'entraînant pas de cytotoxicité.

L'essai a été conduit en duplicate avec :
Lot 1 : épidermes témoins ne recevant pas de produit
Lot 2 : épidermes témoins positifs recevant l'acide kojique, 2%
Lot 3 : épidermes traités recevant crème PXTS + 0,1% AK205
Lot 4 : épidermes traités recevant crème PXTS + 0,5% AK205
AK205 = lyophilisat de cellules dédifférenciées de Criste Marine obtenu selon les Exemples 1 et 2.

Les épidermes fixés dans une solution formaldéhyde 10% ont été inclus dans des blocs en paraffine. Les coupes vert icales de 4 microns ont été colorées à l'hématoxyline/éosine et photographiées sous un microscope optique.

Les cultures doivent présenter des couches cellulaires basales, spineuses, granuleuses et cornées intactes, orthokératosiques, et la stratification épidermique doit être régulière et normale. Les cellules de la couche basale doivent être polarisées verticalement. De nombreux grains de kératohyaline doivent être visibles (en violet) dans la couche granuleuse juste sous la couche cornée.

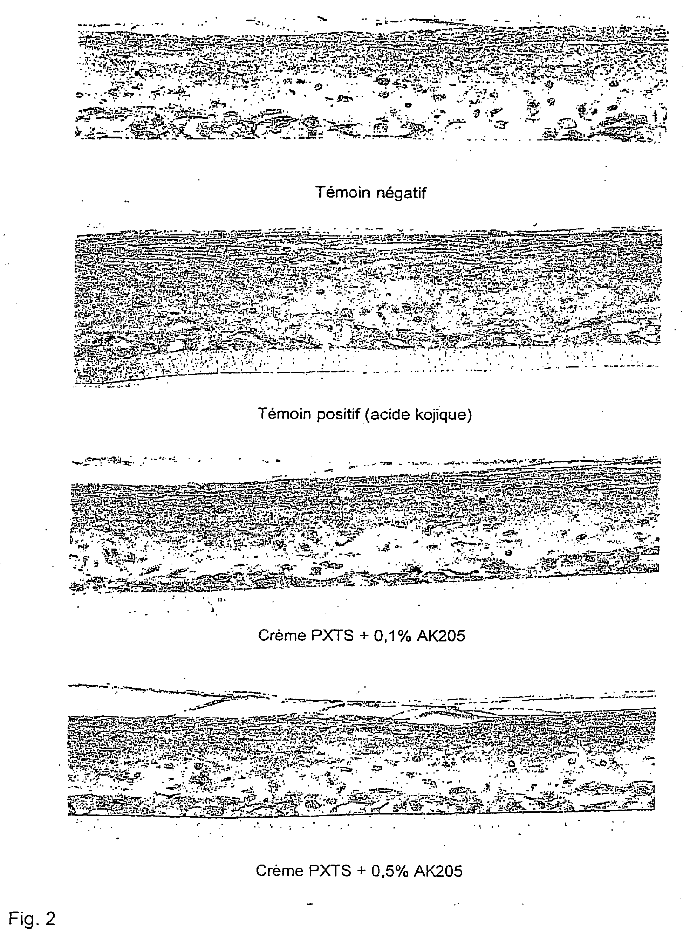

Les produits, crème PXTS+0,1% AK205 et crème PXTS+0,5% AK205 déposés à raison de 2 µL par cm², sur des épidermes reconstitués traités pendant 24 heures, comparativement à des épidermes témoins, n'ont induit aucune toxicité. Les images histologiques, après coloration hématoxyline/éosine, d'épidermes traités sont comparables à celles des épidermes témoins (cf. Figure 2).

### Exemple 4 : Evaluation de l'effet dépigmentant d'une préparation cosmétique contenant un lyophilisat de cellules dédifférenciées de Criste Marine sur des épidermes reconstitués SKINETHIC^{®} intégrant des mélanocytes :

### 1- Protocole expérimental

Des kératinocytes d'origine humaine et des mélanocytes sont ensemencés sur des filtres en polycarbonate de 0,63 cm² dans un milieu défini (MCDB 153 modifié) et supplémenté. Les cellules sont cultivées pendant 10 jours à l'interface air/liquide, le milieu de culture étant changé tous les jours.

Les épidermes de type VI (= négroïde) ainsi formés ont été utilisés à partir du 10ème jour de culture.

Tous les tests ont été conduits en duplicate avec :
Lot 1 : épidermes témoins ne recevant pas de produit
Lot 2 : épidermes témoins positifs recevant l'acide kojique, 2%
Lot 3 : épidermes traités recevant crème PXTS + 0,1% AK205
Lot 4 : épidermes traités recevant crème PXTS + 0,5% AK205
AK205 = lyophilisat de cellules dédifférenciées de Criste Marine obtenu selon les Exemples 1 et 2.

L'évaluation de la synthèse de la mélanine (étude qualitative) intracellulaire a été effectuée par spectrométrie à 475 nm après que les cellules ont été mises en suspension puis dissoutes dans du NaOH (1N) et du diméthyl sulfoxyde pendant 30 minutes.

A la fin de la période d'incubation, le milieu de culture a été prélevé puis les épidermes ont été rincés avec du PBS (Phosphate Buffer Saline) et mis au contact du Triton X-100 (Sigma, France) à 1% puis incubés pendant 10 minutes. La réaction enzymatique a été initiée par l'ajout de la L-Dopamine (Sigma, France) à 10 mM dans du PBS dépourvu de Ca²⁺ et de Mg²⁺.
Après 1 heure d'incubation à 37°C à l'abri de la lumière, l'activité de la tyrosinase a été évaluée par la mesure de l'absorption à 475 nm à l'aide d'un spectrophotomètre.

### 2- Résultats

### a) Dosage de la mélanine

Les résultats obtenus sont présentés sous forme de tableau:

| | Absorbance (475 nm) | % |
|---|---|---|
| Témoin négatif | 0,160 ± 0,02 | - |
| Témoin positif | 0,09 ± 0,01 | -44 |
| CREME PXTS+0,1% AK205 | 0,139 ± 0,02 | -13 |
| CREME PXTS+0,5% AK205 | 0,101 ± 0,01 | -37 |

Les résultats obtenus ont révélé que la crème PXTS+0,5% AK205 a entraîné une diminution significative du taux de mélanine au niveau des épidermes reconstitués intégrant des mélanocytes (-37%). La crème PXTS+0,1% AK205 a réduit légèrement ce taux (-13%) comparativement au témoin positif acide kojique 2% (-44%).

### b) Evaluation de l'activité tyrosinasique

Les résultats obtenus sont présentés sous forme de tableau:

| | Absorbance (475 nm) | % |
|---|---|---|
| Témoin négatif | 0,245 ± 0,03 | - |
| Témoin positif | 0,153 ± 0,01 | -38 |
| CREME PXTS+0,1% AK205 | 0,198 ± 0,02 | -19 |
| CREME PXTS+0,5% AK205 | 0,167 ± 0,02 | -32 |

Les résultats obtenus ont révélé que le produit crème PXTS+0,5% AK205 a entraîné une diminution significative de l'activité de la tyrosinase au niveau des épidermes reconstitués intégrant des mélanocytes (-32%) comparativement au témoin positif (-38%). Une légère diminution (-19%) après traitement par le produit crème PXTS+0,1% AK205 a été observée.

En conclusion, dans les conditions expérimentales retenues le produit crème PXTS+0,5% AK205 a présenté une activité dépigmentante nette vis à vis des épidermes reconstituants intégrant des mélanocytes. Une activité plus limitée mais réelle a été observée avec la crème PXTS+0,1% AK205.

### Exemple 5 : Mise en évidence sur épiderme reconstitué SKINETHIC^{®} de l'effet anti-radicalaire d'une préparation cosmétique contenant un lyophilisat de cellules dédifférenciées de Criste Marine :

### 1- Pourquoi doser le malondialdéhyde?

Dans les systèmes biologiques, l'oxygène moléculaire est stable et peu réactif. Il se comporte comme un accepteur d'électrons et sa réduction aboutit à la production d'eau. Mais la réduction incomplète de l'O₂ aboutit à la production de radicaux libres et de métabolites tels que l'anion superoxyde O₂⁻, le radical perhydroxyde HO₂⁻ toxique (en présence de fer ferreux, la réaction peut conduire à OH-, très agressif) ou le peroxyde d'hydrogène H₂O₂.

La superoxyde di smutase (SOD) protège les membranes en dismutant très rapidement O₂⁻ en H₂O₂. L'H₂O₂, relativement stable, est réduit en eau (H₂O) par la catalase et la peroxydase. Ces radicaux libres issus de la réduction incomplète de l'O₂ sont sensibles au niveau des doubles liaisons, ce qui favorise l'apparition d'autres radicaux libres par délocalisation électronique. L'initiation de la réaction en chaîne radicalaire se produit au niveau des acides gras polyinsaturés. La réaction en chaîne se déclenche alors, à l'intérieur de la membrane cellulaire conduisant à la libération du malondialdéhyde (MDA) ainsi que d'autres aldéhydes et alcanes qui sont des produits de dégradation. Ces derniers peuvent être mis en évidence par réaction avec l'acide thiobarbiturique (TBA).

En dosant le MDA, qui est l'un des marqueurs essentiels de la cytotoxicité par les processus oxydatifs et le stress, on dispose alors d'un indice qui renseigne sur l'activité antiradicalaire d'une substance donnée.

### 2- Protocole expérimental :

Des kératinocytes d'origine humaine sont ensemencés sur des filtres en polycarbonate de 0,63 cm² dans un milieu défini (MCDB 153 modifié) et supplémenté. Les cellules sont cultivées pendant 14 jours à l'interface air/liquide, le milieu de culture étant changé tous les deux jours.

Les épidermes ainsi formés ont été utilisés pour la réalisation de l'étude à partir du 17ème jour de culture.

L'essai a été conduit en triplicate après 24 h de contact du produit avec les épidermes:
- Lot 1 : épidermes témoins négatifs ne recevant aucun produit.
- Lot 2 : épidermes traités recevant le produit crème PXTS+0,1% AK205
- Lot 3 : épidermes traités recevant le produit crème PXTS+0,5% AK205
- Lot 4 : épidermes traités recevant le produit lyophilisat de cellules (Criste Marine)

Les produits à étudier ont été appliqués à la surface de chaque épiderme traité, à raison de 2 µL/cm².

### => Extraction du malondialdéhyde

Après 24h de contact du produit avec les épidermes, ces derniers ont été remis en suspension dans:
- 250 µL de tampon Tris 50 mM, pH 8 contenant NaCl 0,1 M ; EDTA 20 mM
- 25 µL de SDS à 7%
- 300 µL de HCl (0,1 N)
- 38 µL d'acide phosphotungstique à 1% dans l'eau
- 300 µL d'acide thiobarbiturique à 0,67% dans l'eau

Après 1 heure d'incubation dans l'obscurité à 50°C et un refroidissement dans l'eau glacée, 300 ml de n-butanol ont été ajoutés dans chaque tube. Ceux-ci ont été centrifugés à 10 000 g à 0°C pendant 10 min. La phase supérieure a été récupérée pour le dosage du MDA.

### => Dosage du malondialdéhyde

Le MDA a été dosé par mesure de la fluorescence après séparation du complexe MDA-TBA par HPLC (Chromatographie Liquide Haute Pression).
- Pompe Bischoff Model 2.200
- Injecteur automatique Alcoot Model 788 autosampler
- Colonne Ultrasep C18 (30 cm x 0,18 cm) 6 mm de porosité - Détecteur de fluorescence, jasco 821-FI

La détection de la fluorescence a été effectuée avec une excitation à 515 nm et une émission à 553 nm. L'éluent utilisé est composé de méthanol:eau, 40:60 (v/v) dont le pH a été ajusté avec du KOH 1 M.

La quantification a été faite par rapport à des standards traités comme les échantillons (0,125 ; 0,25 ; 0,5 et 1 mM) à l'aide d'un logiciel informatique ICS (Pic 3) (Instrumentation, Consommable Service).

### => Dosage des protéines

Le dosage des protéines a été réalisé selon la méthode de BRADFORD. L'augmentation de l'absorbance à 595 nm est proportionnelle à la concentration des protéines déterminées à l'aide d'un spectrophotomètre UNI CAM 8625.

### 3- Résultats

### a) Lipoperoxydation physiologique

Les résultats obtenus sont présentés sous forme de tableau:

| | MDA (µM/mg protéines) | % |
|---|---|---|
| Témoin | 652 ± 31 | - |
| CREME ⁻PXTS+0,1% AK205 | 638 ± 47 | -2 (ns) |
| CREME PXTS+0,5% AK205 | 620 ± 32 | -5 (ns) |
| LYOPHILISAT DE CELLULES | 594 ± 57 | -9 (ns) |

| | | |
|---|---|---|
| ns : non significatif | | |

Les résultats montrent que les produits étudiés n'induisent aucune libération du MDA dans les conditions physiologiques, par rapport au témoin non traité.

### b) Lipoperoxydation provoquée par des UVB

Les résultats obtenus sont présentés sous forme de tableau:

| | MDA (µM/mg protéines) | % |
|---|---|---|
| Témoin | 652 ± 31 | |
| UVB (150 mJ/cm²) | 832 ± 63 | +28* |
| CREME PXTS+0,1% AK205 + UVB (150 mJ/cm²) | 660 ± 51 | -21** |
| CREME PXTS+0,5% AK205 + UVB (150 mJ/cm²) | 625 ± 42 | -25** |
| LYOPHILISAT DE CELLULES + UVB (150 mJ/cm²) | 602 ± 37 | -28** |

| | | |
|---|---|---|
| * par rapport au témoin négatif ** par rapport au témoin positif irradié UVB | | |

Les résultats obtenus ont révélé une protection significative des produits, crème PXTS+0,1% AK205, crème PXTS+0,5% AK205 et lyophilisat de cellules (Criste Marine), appliqués à la surface de l'épiderme reconstitué SKINETHIC^{®}, vis-à-vis de la lipoperoxydation provoquée par les rayons ultraviolets B (150 mJ/cm²) .

Le pourcentage de réduction de la production de MDA est de -21, -25 et -28% respectivement pour crème PXTS+0,1% AK205, crème PXTS+0,5% AK205 et le produit lyophilisat de cellules (Criste Marine) en comparaison avec les épidermes irradiés.

L'irradiation WB (témoin positif) ayant provoqué +28% d'augmentation de la production de MDA, l'application des produits crème PXTS+0,1% AK205, crème PXTS+0,5% AK205 et produit lyophilisat de cellules (Criste Marine) avant l'irradiation a permis de maintenir la production de MDA à son niveau physiologique.

### Exemple 6 : Mise en évidence sur épiderme reconstitué SKINETHIC^{®} de l'effet stimulant d'une réparation cosmétique contenant un lyophilisat de cellules dédifférenciées de Criste Marine :

### 1- Critères d'évaluation de la stimulation

La culture cellulaire des kératinocytes humains a permis de décrire plus en détails les effets spécifiques des dérivés de la vitamine A sur les marqueurs de la différenciation graduée de l'épiderme : l'expression de KI-67 dans la couche supra-basale est stimulée, l'expression de kératines typiques de l'hyperprolifération épidermique (K19, K13) est induite, il y a perte de la polarité des cellules de la couche basale, tandis que la synthèse de la filaggrine, protéine responsable de l'empaquetage des kératines dans la couche cornée, est inhibée. Les grains de kératohyaline, situés dans la couche granuleuse et riches en filaggrine, disparaissent en 24h en présence d'acide rétinoïque, 0,05% (acide de la vitamine A) dans le milieu de culture. Les rétinoïdes induisent donc globalement une stimulation de la prolifération et une inhibition de la différenciation épidermiques. (Rosdy, M. et al., In Vitro Toxicology, Vol.10 n°1, p. 39-47, 1997, "Retinoic acid inhibits epidermal differentiation when applied topically on the stratum corneum of epidermis formed in vitro by human keratinocytes grown on defined medium")

La filaggrine et la protéine KI-67 peuvent donc être utilisés comme marqueurs de la différenciation épidermique.

### 2- Etude de la différenciation épidermique :

### • Protocole

Des kératinocytes d'origine humaine sont ensemencés sur des filtres en polycarbonate de 0,63 cm² dans un milieu défini (MCDB 153 modifié) et supplémenté. Les cellules sont cultivées pendant 14 jours à l'interface air/liquide, le milieu de culture étant changé tous les deux jours.

Les épidermes ainsi formés ont été utilisés pour la réalisation de l'étude à partir du 17ème jour de culture.

L'essai est conduit en triplicate après 24 heures de contact des produits avec les épidermes :
- Lot 1 : épidermes témoins négatifs ne recevant aucun produit.
- Lot 2 : épidermes traités recevant le produit crème PXTS+0,1% AK205
- Lot 3 : épidermes traités recevant le produit crème PXTS+0,5% AK205
- Lot 4 : épidermes traités recevant le produit lyophilisat de cellules (Criste Marine)

Les épidermes témoins ne recevant aucun produit et les épidermes traités recevant les produits à l'étude pendant 24 heures de contact, ont été congelés à -80°C. Après inclusion en blocs de paraffine, ces épidermes ont été coupés puis traités en immunohistochimie.

Cette réaction a été réalisée avec un anticorps monoclonal recombinant de la filaggrine.

### • Résultats : inhibition de la différenciation des cellules épidermiques

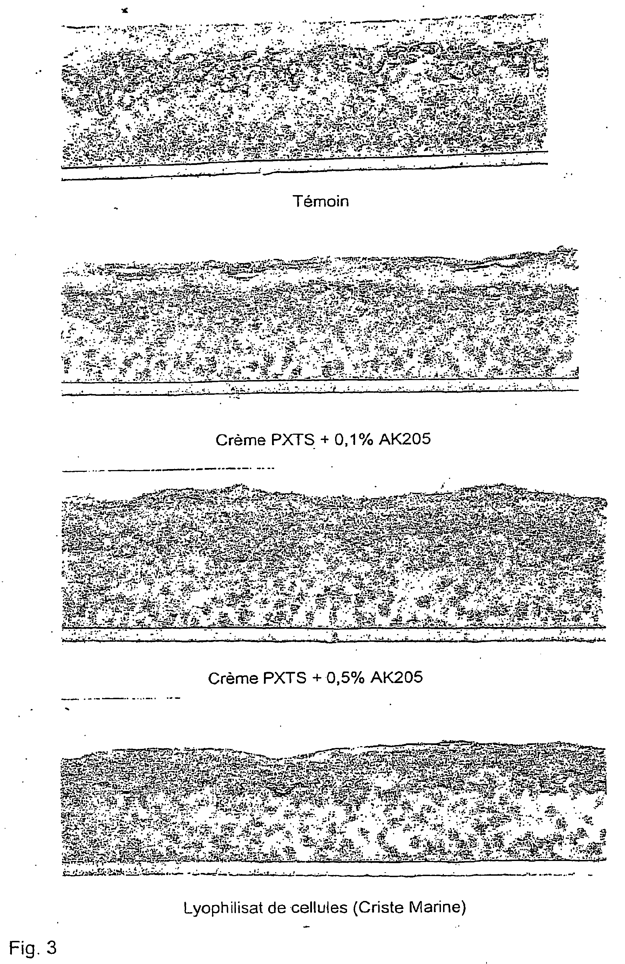

L'observation comparative des épidermes reconstitués témoins et traités avec les produits, crème PXTS+0,1% AK205, crème PXTS+0,5% AK205 et lyophilisat de cellules (Criste Marine), a révélé une différence de densité de marquage de la filaggrine au niveau de la couche granuleuse (cf. Figure 3) .
En effet, dans les conditions physiologiques, le traitement des épidermes par:
- crème PXTS+0,1% AK205: a induit une nette diminution de la différenciation épidermique se traduisant par la nette diminution du marquage de la filaggrine en comparaison avec les témoins non traités,
- crème PXTS+0,5% AK205: a induit une nette diminution de la différenciation épidermique se traduisant par la nette diminution du marquage de la filaggrine en comparaison avec les témoins non traités,
- lyophilisat de cellules (Criste Marine): a induit une diminution significative de la différenciation épidermique se traduisant par la nette diminution du marquage de la filaggrine en comparaison avec le témoin non traité.

### 3- Etude de l'activité prolifératrice des cellules de la couche basale des épidermes

### • Protocole

Des kératinocytes d'origine humaine sont ensemencés sur des filtres en polycarbonate de 0,63 cm² dans un milieu défini (MCDB 153 modifié) et supplémenté. Les cellules sont cultivées pendant 14 jours à l'interface air/liquide, le milieu de culture étant changé tous les deux jours.

Les épidermes ainsi formés ont été utilisés pour la réalisation de l'étude à partir du 17ème jour de culture.

L'activité des produits a été révélée par un marquage immunohistochimique.

L'essai est conduit en triplicate après 24 heures de contact du produit avec les épidermes :
- Lot 1 : épidermes témoins négatifs ne recevant aucun produit.
- Lot 2 : épidermes traités recevant le produit crème PXTS+0,1% AK205)
- Lot 3 : épidermes traités recevant le produit crème PXTS+0,5% AK205
- Lot 4 : épidermes traités recevant le produit lyophilisat de cellules (Criste Marine)

Les épidermes témoins ne recevant aucun produit et les épidermes traités recevant les produits à l'étude pendant 24 heures de contact, ont été fixés dans le formaldéhyde 10%. Après inclusion en blocs de paraffine, ces épidermes ont été coupés puis traités en immunohistochimie.

Cette réaction a été réalisée avec l'anticorps MIB1 (Immunotech), peptide recombinant de l'antigène nucléaire KI-67.

La révélation a été faite par la méthode péroxydase-antipéroxydase après démasquage antigénique par pré-traitement à la chaleur.

Le marquage par chromogène DAB révèle en brun les sites nucléaires KI-67 de la fraction des cellules en croissance exprimée en phases:
=> G1 et S = phases de latence et de synthèse de la cellule
=> G2 = phase de dédoublement des constituants cellulaires
=> M = mitose

L'index mitotique a été évalué par comptage des sites nucléaires colorés, à raison de 10 champs par lame au microscope optique, grossissement x 250, comparativement aux coupes d'épiderme témoins.

### • Résultats : stimulation de la prolifération des cellules épidermiques

Les résultats obtenus sont présentés sous forme de tableau:

| | Moyenne du nombre de noyaux colorés par champ de comptage au microscope |
|---|---|
| Témoin | 7 ± 2 |
| CREME PXTS+0,1% AK205 | 8 ± 2 |
| CREME PXTS+0,5% AK205 | 12 ± 2 |
| LYOPHILISAT DE CELLULES | 14 ± 3 |

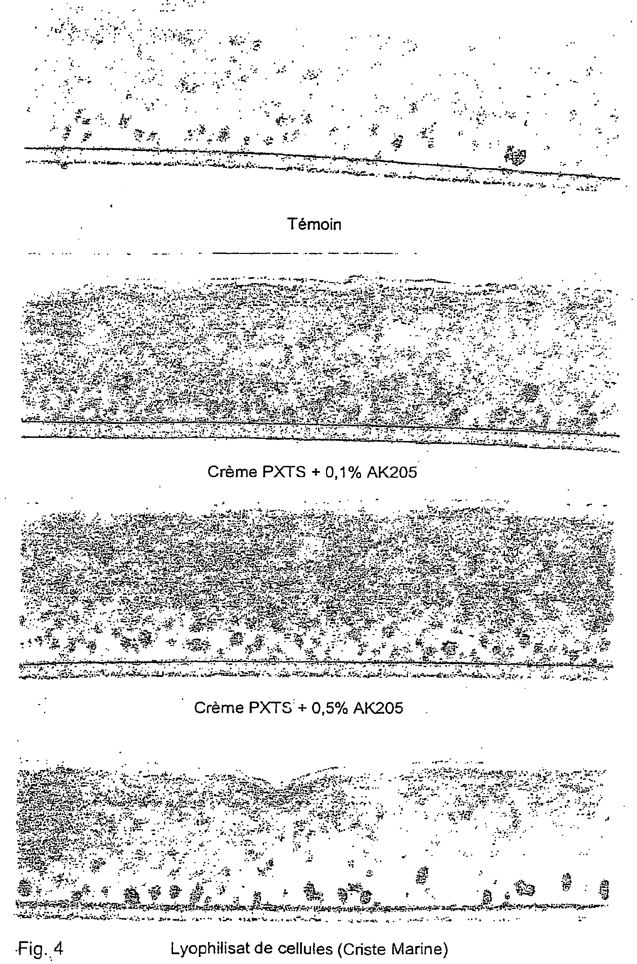

Le comptage des sites nucléaires colorés au brun de tous les échantillons, après traitement immunohistochimique, a révélé que le produit:
- crème PXTS+0,1% AK205 : a entraîné une faible augmentation mais significative de la multiplication des cellules de la couche basale. Le nombre de sites nucléaires colorés est comparable a celui des témoins non traités (cf. Figure 4).
- crème PXTS+0,5% AK205 : a entraîné une faible augmentation mais significative de la multiplication des cellules de la couche basale en comparaison avec le témoin non traité. Le nombre de sites nucléaires colorés est supérieur a celui des témoins non traités (cf. Figure 4).
- lyophilisat de cellules (Criste Marine): a entraîné une augmentation significative de la multiplication des cellules de la couche basale en comparaison avec le témoin non traité. Le nombre de sites nucléaires colorés est largement supérieur a celui des témoins non traités (cf. Figure 4).

## Revendications

1. Utilisation dans une composition cosmétique d'au moins un lyophilisat de cellules végétales dédifférenciées, lesquelles sont des cellules de plantes halophiles, pour dépigmenter et/ou éclaircir et protéger l'épiderme.

2. Utilisation d'au moins un lyophilisat de cellules végétales dédifférenciées, lesquelles sont des cellules de plantes halophiles, pour la préparation d'une composition pharmaceutique destinée à une utilisation pour dépigmenter et/ou éclaircir, protéger et régénérer l'épiderme.

3. Utilisation selon la revendication 1 ou 2, **caractérisée en ce que** les cellules végétales dédifférenciées sont obtenues par culture in vitro.

4. Utilisation selon la revendication 3, **caractérisée en ce que** les cellules végétales dédifférenciées obtenues par culture in vitro sont des lignées.

5. Utilisation selon l'une quelconque des revendications 1 à 4, **caractérisée en ce que** la plante halophile est la Criste Marine.

6. Utilisation selon l'une quelconque des revendications 2 à 5, **caractérisée en ce que** le lyophilisat dépigmente et/ou éclaircit l'épiderme en bloquant l'activité de la tyrosinase.

7. Utilisation selon l'une quelconque des revendications 1 à 6, **caractérisée en ce que** le lyophilisat protège l'épiderme par un effet anti-radicalaire.

8. Utilisation selon l'une quelconque des revendications 2 à 5, **caractérisée en ce que** le lyophilisat régénère l'épiderme par un effet stimulant la prolifération des cellules de la couche basale de l'épiderme.

9. Utilisation selon l'une quelconque des revendications 2 à 5, **caractérisée en ce que** le lyophilisat régénère l'épiderme par un effet inhibant la différenciation épidermique.

10. Composition cosmétique ou pharmaceutique à usage topique **caractérisée en ce qu'**elle comprend, dans une base physiologiquement acceptable, au moins un lyophilisat tel que décrit selon l'une quelconque des revendications 1 à 9.

11. Composition cosmétique ou pharmaceutique à usage topique **caractérisée en ce qu'**elle comprend, dans une base physiologiquement acceptable, de 0,05% à 2% d'au moins un lyophilisat tel que décrit selon l'une quelconque des revendications 1 à 9.

12. Composition cosmétique ou pharmaceutique à usage topique **caractérisée en ce qu'**elle comprend, dans une base physiologiquement acceptable, de 0,1% à 1% d'au moins un lyophilisat tel que décrit selon l'une quelconque des revendications 1 à 9.

13. Composition cosmétique ou pharmaceutique à usage topique **caractérisée en ce qu'**elle comprend, dans une base physiologiquement acceptable, 0,5% d'au moins un lyophilisat tel que décrit selon l'une quelconque des revendications 1 à 9.

14. Composition selon l'une quelconque des revendications 10 à 13, destinée à rajeunir l'aspect de la peau.

15. Composition selon l'une quelconque des revendications 10 à 13, destinée au traitement des taches pigmentaires.

16. Composition selon l'une quelconque des revendications 10 à 13, destinée à éclaircir les peaux noires ou asiatiques.

## Claims

1. Use, in a cosmetic composition, of at least one lyophilizate of undifferentiated plant cells, which are cells of halophilic plants, for depigmenting and/or lightening and protecting the epidermis.

2. Use of at least one lyophilizate of undifferentiated plant cells, which are cells of halophilic plants, for the preparation of a pharmaceutical composition intended for use for depigmenting and/or lightening, protecting and regenerating the epidermis.

3. Use according to claim 1 or 2, **characterized in that** the undifferentiated plant cells are obtained by growing in vitro.

4. Use according to claim 3, **characterized in that** the undifferentiated plant cells obtained by growing *in vitro* are cell lines.

5. Use according to any one of claims 1 to 4, **characterized in that** the halophilic plant is sea fennel.

6. Use according to any one of claims 2 to 5, **characterized in that** the lyophilizate depigments and/or lightens the epidermis by blocking the activity of tyrosinase.

7. Use according to any one of claims 1 to 6, **characterized in that** the lyophilizate protects the epidermis via a free-radical-scavenging effect.

8. Use according to any one of claims 2 to 5, **characterized in that** the lyophilizate regenerates the epidermis via a stimulatory effect on the proliferation of cells of the basal layer of the epidermis.

9. Use according to any one of claims 2 to 5, **characterized in that** the lyophilizate regenerates the epidermis via an inhibitory effect on epidermal differentiation.

10. A cosmetic or pharmaceutical composition for topical use, **characterized in that** it comprises, in a physiologically acceptable base, at least one lyophilizate as described according to any one of claims 1 to 9.

11. A cosmetic or pharmaceutical composition for topical use, **characterized in that** it comprises, in a physiologically acceptable base, from 0.05% to 2% of at least one lyophilizate as described according to any one of claims 1 to 9.

12. A cosmetic or pharmaceutical composition for topical use, **characterized in that** it comprises, in a physiologically acceptable base, from 0.1% to 1% of at least one lyophilizate as described according to any one of claims 1 to 9.

13. A cosmetic or pharmaceutical composition for topical use, **characterized in that** it comprises, in a physiologically acceptable base, 0.5% of at least one lyophilizate as described according to any one of claims 1 to 9.

14. A composition according to any one of claims 10 to 13, for rejuvenating the appearance of the skin.

15. A composition according to any one of claims 10 to 13, for treating pigmentary marks.

16. A composition according to any one of claims 10 to 13, for lightening black or asiatic skin.

## Patentansprüche

1. Verwendung, in einer kosmetischen Zusammensetzung, wenigstens eines Lyophilisats aus entdifferenzierten Pflanzenzellen, die Zellen von halophilen Pflanzen sind, um die Haut zu depigmentieren und/oder zu bleichen und zu schützen.

2. Verwendung wenigstens eines Lyophilisats aus entdifferenzierten Pflanzenzellen, die Zellen von halophilen Pflanzen sind, um eine pharmazeutische Zusammensetzung herzustellen, die bestimmt ist für eine Anwendung zum Depigmentieren und/oder Bleichen, Schützen und Regenerieren der Haut.

3. Verwendung nach Anspruch 1 oder 2, **dadurch gekennzeichnet, dass** die entdifferenzierten Pflanzenzellen mittels In-vitro-Kultur gewonnen werden.

4. Verwendung nach Anspruch 3, **dadurch gekennzeichnet, dass** die mittels In-Vitro-Kultur gewonnenen entdifferenzierten Pflanzenzellen Linien sind.

5. Verwendung nach einem der Ansprüche 1 bis 4, **dadurch gekennzeichnet, dass** die halophile Pflanze die Strandfenchel ist.

6. Verwendung nach einem der Ansprüche 2 bis 5, **dadurch gekennzeichnet, dass** das Lyophilisat die Haut depigmentiert und/oder bleicht, indem es die Aktivität der Tyrosinase blockiert.

7. Verwendung nach einem der Ansprüche 1 bis 6, **dadurch gekennzeichnet, dass** das Lyophilisat die Haut durch einen Antiradikaleneffekt schützt.

8. Verwendung nach einem der Ansprüche 2 bis 5, **dadurch gekennzeichnet, dass** das Lyophilisat die Haut durch einen die Proliferation der Zellen der Basalschicht der Haut stimulierenden Effekt regeneriert.

9. Verwendung nach einem der Ansprüche 2 bis 5, **dadurch gekennzeichnet, dass** das Lyophilisat die Haut durch einen die Hautdifferenzierung hemmenden Effekt regeneriert.

10. Kosmetische oder pharmazeutische Zusammensetzung zum topischen Gebrauch, **dadurch gekennzeichnet, dass** sie in einem physiologisch akzeptablen Grundstoff wenigstens ein Lyophilisat wie beschrieben nach einem der Ansprüche 1 bis 9 enthält.

11. Kosmetische oder pharmazeutische Zusammensetzung zum topischen Gebrauch, **dadurch gekennzeichnet, dass** sie in einem physiologisch akzeptablen Grundstoff 0,05% bis 2% wenigstens eines Lyophilisats wie beschrieben nach einem der Ansprüche 1 bis 9 enthält.

12. Kosmetische oder pharmazeutische Zusammensetzung zum topischen Gebrauch, **dadurch gekennzeichnet, dass** sie in einem physiologisch akzeptablen Grundstoff 0,1% bis 1% wenigstens eines Lyophilisats wie beschrieben nach einem der Ansprüche 1 bis 9 enthält.

13. Kosmetische oder pharmazeutische Zusammensetzung zum topischen Gebrauch, **dadurch gekennzeichnet, dass** sie in einem physiologisch akzeptablen Grundstoff 0,5% wenigstens eines Lyophilisats wie beschrieben nach einem der Ansprüche 1 bis 9 enthält.

14. Zusammensetzung nach einem der Ansprüche 10 bis 13, bestimmt zur Verjüngung des Aussehens der Haut.

15. Zusammensetzung nach einem der Ansprüche 10 bis 13, bestimmt zum Behandeln von Pigmentflecken.

16. Zusammensetzung nach einem der Ansprüche 10 bis 13, bestimmt zum Bleichen von schwarzer oder asiatischer Haut.
